Europäisches Patentamt

European Patent Office

Office européen des brevets

(19)

(11) Veröffentlichungsnummer : **0 154 002**
**B1**

(12) # EUROPÄISCHE PATENTSCHRIFT

(45) Veröffentlichungstag der Patentschrift :
07.06.89

(21) Anmeldenummer : 84114453.8

(22) Anmeldetag : 29.11.84

(51) Int. Cl.⁴ : **A 61 B 5/14**, **A 61 M 1/00**,
**A 61 M 25/00**, **G 01 N 1/18**

(54) **Vorrichtung zum Abnehmen von Blutproben für die Diagnose von Körperfunktionen.**

(30) Priorität : 04.02.84 DE 3403957

(43) Veröffentlichungstag der Anmeldung :
11.09.85 Patentblatt 85/37

(45) Bekanntmachung des Hinweises auf die Patenterteilung : 07.06.89 Patentblatt 89/23

(84) Benannte Vertragsstaaten :
AT CH DE FR GB IT LI NL SE

(56) Entgegenhaltungen :
EP—A— 0 107 579
DE—A— 1 541 110
DE—B— 2 803 345
US—A— 3 494 351
US—A— 3 848 581
US—A— 3 908 657

(73) Patentinhaber : **Ferring Biotechnik GmbH**
**Wittland 11-13**
**D-2300 Kiel 1 (DE)**

(72) Erfinder : **Paulsen, Otto, Dr.**
**Wittland 11**
**D-2300 Kiel 1 (DE)**
Erfinder : **Harm Kölln**
**Fallreep 43**
**2300 Kiel 17 (DE)**

(74) Vertreter : **Türk, Gille, Hrabal**
**Bruckner Strasse 20**
**D-4000 Düsseldorf 13 (DE)**

EP 0 154 002 B1

## Beschreibung

Die Erfindung betrifft eine Vorrichtung zum Abnehmen von Blutproben in zeitlichen Abständen nacheinander für die Diagnose von Körperfunktionen eines Patienten durch Analysieren einzelner Blutproben.

Für die Diagnose von Körperfunktionen eines Patienten ist es bekannt, demselben nacheinander mehrere Blutproben abzunehmen und diese einzelnen Blutproben zu analysieren, um danach zu bestimmen, ob und gegebenenfalls welche Medikamente dem Patienten verabreicht werden müssen. Beispielsweise entnimmt man dem Patienten eine Blutprobe bei nüchternem Magen und weitere Blutproben nach dem Frühstück und/oder nach körperlicher Belastung. Die zeitlichen Abstände zwischen der Abnahme der einzelnen Blutproben sind dabei sehr unterschiedlich und auch ungenau, so daß die Analyse der Blutproben oft kein exaktes Bild der Körperfunktionen des Patienten liefern kann. Ein weiterer Nachteil besteht darin, daß im allgemeinen nur zwei oder höchstens drei Blutproben entnommen und analysiert werden, worauf ebenfalls zurückzuführen ist, daß die Körperfunktionen des Patienten durch eine derartige Blutanalyse nur ungenau zu erfassen sind. Dementsprechend läßt sich die notwendige Therapie auch nicht mit ausreichender Genauigkeit festlegen.

In WO 84/04033, deren Inhalt gilt als Stand der Technik gemäß Art. 5413) EPÜ, ist eine Vorrichtung zum Abnehmen von Blutproben in zeitlichen Abständen nacheinander für die Diagnose von Körperfunktionen eines Patienten offenbart, die funkferngesteuert arbeitet und entsprechend nach einem vorgegebenen Programm gesandten Funksignalen so gesteuert wird, daß abwechselnd nacheinander Blutproben und inertes Trennmittel in ein oder mehrere einzelne Behältnisse eingefüllt werden. Der Patient muß sich dabei stets im Sendebereich des Funksteuergerätes bzw. Senders aufhalten, was seinen Aktionsradius begrenzt und den weiteren Nachteil hat, daß der Patient auf seine Position achten muß, wodurch sich bei ambulanter Behandlung sein Blutbild verändern kann und dementsprechend die analysierten Blutproben ein falsches Bild seiner Körperfunktionen geben können. Diese Vorrichtung weist ein ständig mit dem Patienten zu verbindendes Katheter auf, eine an diesen Katheter angeschlossenen, nach einem vorgegebenen Programm automatisch ein- und auszuschaltenden Pumpe und wenigstens ein mit dem Katheter über ein umschaltbares Steuerventil verbundenes Sammelbehältnis für die abgenommenen Blutproben auf, wobei das Steuerventil in steuerbarer Folge das Sammelbehältnis abwechselnd mit dem Katheter und einem Vorratsbehältnis für inertes Trennmittel und/oder Antigerinnungsmittel verbindet.

Der Erfindung liegt die Aufgabe zugrunde, die Abnahme von Blutproben für die Diagnose der Körperfunktionen des Patienten für den Patienten zu erleichtern, indem er sich weitgehend ungehindert bewegen kann und dabei das Sammeln der Blutproben schon durchzuführen, so daß sich durch die anschließende Analyse der entnommenen Blutproben ein sehr viel genaueres Bild der Körperfunktionen des Patienten als bisher möglich ergibt.

Diese Aufgabe wird erfindungsgemäß durch eine Vorrichtung mit den Merkmalen des Anspruchs 1 gelöst. Vorteilhafte Ausgestaltungen ergeben sich aus den Merkmalen der Unteransprüche.

Die erfindungsgemäße Vorrichtung weist einen ständig mit dem Patienten zu verbindenden Katheter, einen an diesen angeschlossenen flexiblen Schlauch, eine nach einem vorgegebenen Programm automatisch ein- und ausschaltbare Rollenpumpe, welche auf den flexiblen Schlauch einwirkt, sowie einen individuell programmierbaren Mikrocomputer auf, der die Rollenpumpe und ein Steuerventil steuert, welches in steuerbarer Folge die Behältnisse für die abgenommenen Blutproben abwechselnd mit dem Katheter und einem Vorratsbehälter für inertes Trennmittel und/oder Antigerinnungsmittel verbindet. Die Vorrichtung ist tragbar ausgebildet und kann somit am Patienten für die Dauer der Blutprobenentnahme festgelegt werden. Der Arzt braucht zu Beginn lediglich den Katheter einzusetzen und den Programmablauf für die Steuerung einzustellen und auszulösen. Die Blutprobenentnahme erfolgt automatisch in der gewünschten Reihenfolge und für die gewünschte Zeitdauer, wobei sich der Patient frei bewegen kann. Nach Beendigung der Entnahmeperiode kehrt der Patient zum behandelnden Arzt zurück, der den Katheter löst und die Vorrichtung vom Patienten abnimmt.

Dadurch, daß die dem Patienten entnommenen Blutproben zunächst gesammelt und dabei voneinander getrennt gehalten werden, bis die für die Analyse gewünschte Anzahl von Blutproben abgenommen worden ist, ist eine ambulante Blutprobenabnahme möglich, d. h. der Patient kann sich während der Abnahme der Blutproben frei bewegen, weil erst am Ende der Abnahme aller Blutproben diese dem behandelnden Arzt abgegeben werden müssen. Bisher ist eine Blutentnahme in genau vorgegebenen Zeitabständen über einen ganzen Tag verteilt praktisch nur bei stationärem Klinikaufenthalt möglich. Ein Klinikaufenthalt kann aber nicht die natürlichen Verhältnisse des Patienten wiedergeben, da die üblichen Belastungen bei körperlicher Arbeit oder auch normaler Schreibtischarbeit fehlen.

Damit die dem Patienten nacheinander entnommenen einzelnen Blutproben bis zur Analyse nicht gerinnen, wird ihnen zweckmäßig ein Antigerinnungsmittel beigegeben. Beispielsweise wird das Blut unmittelbar nach der Entnahme mit dem durch ein Ventil zugeführten Antigerinnungsmittel versetzt. Eine andere Möglichkeit besteht darin, ein Antigerinnungsmittel mit einem Trennmittel, z. B. Silikonöl, zu emulgieren. Dadurch wird die Wand des die Blutproben aufnehmenden Behältnisses so mit dem Antigerinnungsmittel benetzt, daß das eingefüllte Blut nicht gerinnt.

Damit die einzelnen Blutproben mit Sicherheit voneinander getrennt in einem Behältnis gesammelt und aufbewahrt werden können, wird zwischen den einzelnen Blutproben in das Sammelbehältnis ein inertes Trennmittel, beispielsweise auf Silikonbasis, aber gegebenenfalls auch Gas oder Luft, eingegeben, das zwischen den einzelnen Blutproben eine Trennschicht bildet, die ein Vermischen der Blutproben mit Sicherheit verhindert. Alternativ kann man die einzelnen Blutproben auch in einzelnen Behältnissen sammeln wenn keine allzu große Anzahl von Blutproben für die Analyse benötigt wird. Die gesammelten Blutproben können dann zur Analyse in das Labor gegeben werden.

Jedes Behältnis kann als flexibler Schlauch ausgebildet und auf eine Rolle aufwickelbar sein, damit eine größere Schlauchlänge für eine Vielzahl von Blutproben auf möglichst kleinem Raum untergebracht werden kann. Weiterhin kann das Sammelbehältnis aus durchsichtigem flexiblem Material bestehen, so daß unter Umständen die Blutproben für die Analyse noch nicht einmal aus dem Sammelbehältnis entnommen zu werden brauchen.

Im allgemeinen kommt man mit einer Zugabeeinrichtung für ein Trennmittel aus. Es sind aber Fälle vorstellbar, in denen eine zweite Zugabeeinrichtung für das Antigerinnungsmittel notwendig ist, nämlich wenn das Antigerinnungsmittel mit dem zu untersuchenden Stoff reagiert und man ein anderes Gerinnungsmittel verwenden muß, das sich mit dem Trennmittel wie Silikonöl nicht emulgieren läßt. Dann kann die erfindungsgemäße Vorrichtung zusätzlich zur Zugabeeinrichtung für Trennmittel zwischen die einzelnen Blutproben mit einer weiteren Zugebeeinrichtung für ein Antigerinnungsmittel versehen sein, wobei diese Einrichtungen jeweils eine Pumpe wie eine Rollenpumpe einschließen können.

Werden die nacheinander entnommenen einzelnen Blutproben jeweils in einem gesonderten Behältnis aufgefangen und bis zur Analyse aufbewahrt, benötigt man kein Trennmittel, so daß eine entsprechende Zugabeeinrichtung nicht notwendig ist. Aber auch in diesem Fall ist es erforderlich, ein Antigerinnungsmittel in die einzelnen Behältnisse einzugeben, damit die Blutproben bis zur Analyse nicht gerinnen. Hierzu ist beispielsweise eine Zugabeeinrichtung vorzusehen, welche unmittelbar vor oder während des Einfüllens einer Blutprobe in das Behältnis das Antigerinnungsmittel eingibt.

Eine andere Möglichkeit besteht darin, die Innenwände der einzelnen Behältnisse vorher mit einem Antigerinnungsmittel zu präparieren oder das Antigerinnungsmittel von vornherein in ausreichender Menge in den betreffenden Behältnissen unterzubringen. Dann kann eine gesonderte Zugabeeinrichtung entfallen.

Die Zugabeeinrichtung umfaßt vorzugsweise ein umschaltbares Steuerventil, das in steuerbarer Folge das betreffende Behältnis abwechselnd mit dem Katheter und einem Vorratsbehälter für inertes Trennmittel und/oder Antigerinnungsmittel verbindet. Dieses Steuerventil kann gemeinsam mit der Rollenpumpe von einem Mikrocomputer nach vorgegebenem Programm umgeschaltet werden. Es ist beispielsweise ein Dreiwege-Ventil, beispielsweise ein Schieberventil oder auch ein Dreiwege-Hahn.

Im Falle eines Schieberventils ist der Schieber des Ventils beispielsweise elektromagnetisch steuerbar, jedoch sind auch andere Steuermöglichkeiten denkbar, beispielsweise mit Hilfe von Druckluft, ggfs. kombiniert mit einer Rückstellfeder, oder mit Hilfe eines Zahnstangengetriebes.

Auch ist es möglich, daß das umschaltbare Steuerventil einen einzigen Durchgang enthält, der entsprechend der Stellung des Steuerelementes wie des Steuerschiebers den Katheter wahlweise mit einer Anzahl von Behältnissen zur Aufnahme jeweils einzelner Blutproben und einem Behältnis zur Aufnahme von Blutabfällen verbindet. Bei Verwendung eines derartigen Steuerventils entfällt die Notwendigkeit, ein Trennmittel zu verwenden, weil die einzelnen Blutproben in voneinander getrennte Behältnisse eingefüllt und in diesen aufbewahrt werden. Die zwischen der Entnahme einzelner Blutproben in den Katheter und der Leitung zum Steuerventil einschließlich dem Steuerventil verbleibenden Blutmengen werden jeweils zu Beginn der Entnahme einer weiteren Blutprobe in ein Sammelbehältnis als Abfall eingeleitet, bevor die frisch entnommene Blutprobe, welche den Rest der vorher entnommenen Blutprobe durch den Katheter und das Steuerventil in das Abfallbehältnis geschoben hat, durch danach erfolgendes Umschalten des Steuerventils in das dafür vorgesehene Behältnis geleitet wird.

Eine derartige vorrichtung ist für die Abnahme von Blutproben in begrenzter Anzahl zweckmäßig. Die Betätigung des Steuerschiebers erfolgt beispielsweise über ein Zahnstangengetriebe.

Die Erfindung ist auch dazu geeignet, den Anstieg des Blutspiegels bestimmter körpereigener Stoffe nach Verabreichung von Arzneimitteln zu messen, insbesondere für wissenschaftliche Untersuchungen. So haben die meisten körpereigenen Hormone einen unterschiedlichen Ausschüttungsrhythmus, den man bisher nur bei wenigen Hormonen aufgrund der schwierigen Blutentnahme kennt. Die vorliegende Erfindung macht es möglich, den Ausscheidungsrhythmus aller menschlichen Hormone mit vertretbarem Aufwand zu bestimmen.

In der Zeichnung ist ein Ausführungsbeispiel einer erfindungsgemäßen Vorrichtung zum Abnehmen einer Mehrzahl von Blutproben in einer vorprogrammierbaren Folge schematisch dargestellt, und zwar zeigt

Figur 1 eine Gesamtansicht der Vorrichtung,

Figur 2 Einzelheiten des Sammelgerätes der Vorrichtung,

Figur 3 eine Ausführungsform des Steuerventiles in zwei unterschiedlichen Betriebspositionen,

Figur 4 eine Teilansicht eines schlauchförmigen Sammelbehältnisses mit eingefüllten Blutproben und zwischen diesen befindlicher inerter Trennflüssigkeit und

Figur 5 eine andere Ausführungsform des Steuerventils.

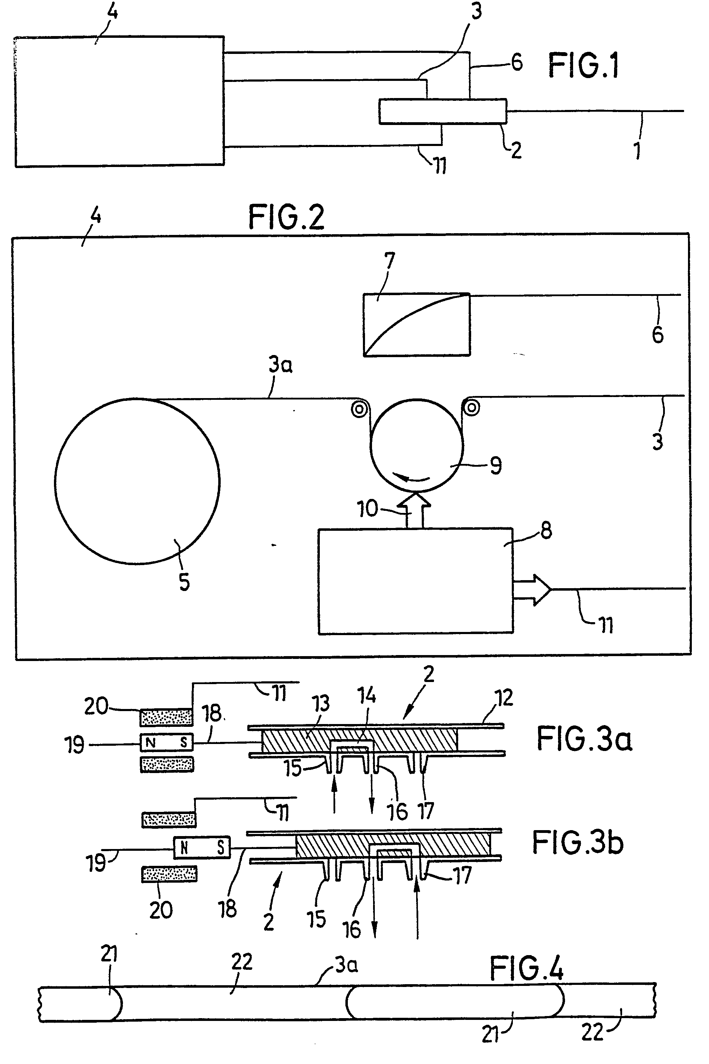

Die Vorrichtung hat gemäß Fig. 1 einen an den Patienten anzuschließenden Katheter (1), der über ein Steuerventil (2) an einen flexiblen Schlauch (3) angeschlossen ist, der in ein Sammelgerät (4) führt und dort als Sammelbehältnis (3a) für Blutproben auf eine Rolle (5) aufgewickelt ist.

An das Ventil (2) ist außerdem ein Schlauch (6) angeschlossen, durch den aus einem Vorratsbehälter (7) eine als Trennmittel dienende inerte Flüssigkeit, beispielsweise Silikonöl, dem Ventil (2) zugeführt und von diesem in den Schlauch (3) geleitet werden kann.

Im Sammelgerät (4) befindet sich ein Mikrocomputer (8), der mit einem nicht dargestellten Tastenfeld und einer Anzeige versehen ist und zum Steuern des Ventils (2) und einer auf den Schlauch (3) einwirkenden Rollenpumpe (9) dient. Der Mikrocomputer (8) ist zu diesem Zweck mit der Rollenpumpe (9) über eine elektrische Leitung (10) und mit dem Ventil (2) über eine weitere elektrische Leitung (11) verbunden. Mit Hilfe des Tastenfeldes und der Anzeige kann man in dem Mikrocomputer ein Programm für die Entnahme von Blutproben in vorgegebenen zeitlichen Intervallen einspeichern. Der Mikrocomputer (8) setzt dann zu den vorgegebenen Zeiten die Rollenpumpe (9) in Bewegung, um dem Patienten durch den Katheter (1) eine Blutprobe zu entnehmen. Das Ventil (2) ist dann so eingestellt, daß der Katheter (1) mit dem Schlauch (3) verbunden ist.

Zum Beenden jeder Blutprobenentnahme wird das Ventil (2) umgeschaltet, um den Schlauch (6) mit dem Schlauch (3) zu verbinden und flüssiges Trennmittel aus dem Vorratsbehälter (7) in den Schlauch (3) und seine das Sammelbehältnis (3a) bildende Verlängerung anzusaugen. Danach wird die Rollenpumpe (9) abgestellt, wenn nicht unmittelbar anschließend eine weitere Blutprobe entnommen werden soll.

Die Rollenpumpe (9) fördert die einzelnen Blutproben und das jeweils dazwischen befindliche Trennmittel in das mehrlagig auf die Rolle (5) aufgewickelte schlauchförmige Sammelbehältnis (3a), das nach Beendigung der Blutprobenentnahme aus dem Sammelgerät (4) herausgenommen wird, um die Blutproben im Labor in üblicher Weise zu analysieren.

Das Ventil (2) ist, wie Fig. 3 zeigt, ein Schieberventil, das in einem Zylinder (12) einen Kolbenschieber (13) aufweist, der einen Kanal (14) enthält, welcher je nach Stellung des Kolbens (13) im Zylinder (12) jeweils zwei von drei Anschlußstutzen (15, 16 und 17) miteinander verbindet. An den Anschlußstutzen (15) ist der Schlauch (6), an den Anschlußstutzen (16) ist der Schlauch (3) und an den Anschlußstutzen (17) der Katheter (1) angeschlossen.

Der Kolbenschieber (13) ist mit einer Kolbenstange (18) versehen, an deren äußeren Ende sich ein Permanentmagnet (19) befindet, der mit einer elektrischen Tauchspule (20) zusammenwirkt. Die Tauchspule (20) wird mittels durch die elektrische Leitung (11) vom Mikrocomputer (8) kommenden Schaltimpulsen gesteuert mit elektrischer Energie versorgt, so daß der Permanentmagnet (19) und damit der Kolbenschieber (13) zwischen zwei Positionen verschiebbar ist, die in Fig. 3a) und Fig. 3b) dargestellt sind.

In der Position gemäß Fig. 3a) verbindet der Kanal (14) die Anschlußstutzen (15 und 16) und damit den Schlauch (6) mit dem Schlauch (3). In der Position gemäß Fig. 3b) verbindet der Kanal (14) die Anschlußstutzen (16 und 17) und damit den Schlauch (3) mit dem Katheter (1). Dementsprechend wird bei laufender Rollenpumpe (9) entweder inerte Trennflüssigkeit aus dem Vorratsbehälter (7) (Fig. 3a) oder Blut aus dem Katheter (1) in den Schlauch (3) eingesaugt (Fig. 3b).

Bei einer derartigen Vorrichtung stammt bei gleichmäßigen zeitlichen Abständen der Blutentnahme der erste Teil einer herangeführten Blutzufuhr noch von der vorher angesaugten Blutentnahme. Um eine sichere Trennung der Blutproben zu erreichen, steuert man deshalb die Entnahme derart, daß jeweils kurz hintereinander voneinander getrennt zwei Blutproben entnommen werden und jeweils nur die zweite, also unvermischte, Blutprobe zur Analyse herangezogen wird. Der Kolbenschieber (13) des Ventils (2) läßt sich relativ einfach verstellen, nämlich durch Umpolung des Elektromagneten (20), durch Einwirkung von Druckluft oder auch durch ein Zahnradgetriebe.

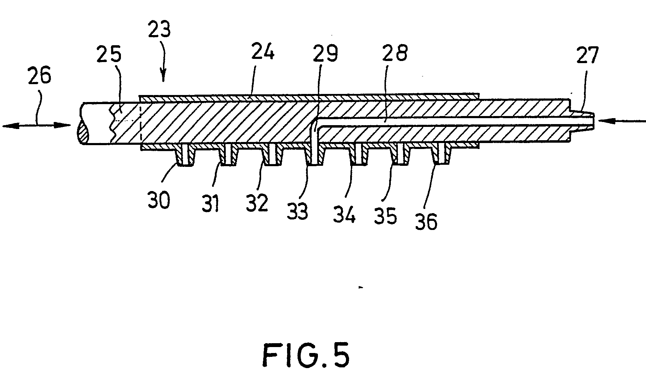

Fig. 4 zeigt, daß die im schlauchförmigen Sammelbehältnis (3a) befindlichen einzelnen Blutproben (21) gegenüber dem Schlauch (3) und dem Sammelbehältnis (3a) eine höhere Oberflächenspannung als die dazwischen befindlichen Trennmittelpuffer (22) aus inerter Flüssigkeit wie Silikonöl aufweisen. Daher hat die Rollenpumpe (9) keinen Einfluß auf die Trennung zwischen den aufeinanderfolgenden Blutproben (21), d. h. auch im Bereich der Rollenpumpe (9) können aufeinanderfolgende Blutproben (21) nicht zusammenlaufen und sich auch nicht mit dem Trennmittel vermischen.

Das in Fig. 5 dargestellte Steuerventil (23) hat einen als Gehäuse dienenden Zylinder (24), in welchem ein Kolbenschieber (25) axial verschiebbar geführt ist. Am — in Fig. 5 gesehen — linken Ende des Kolbenschiebers (25) ist ein Antrieb angeordnet, der zur Vereinfachung der Darstellung weggelassen ist und durch einen Doppelpfeil (26) angedeutet wird. Am gegenüberliegenden Ende des Kolbenschiebers (25) befindet sich ein Anschlußstutzen (27), an den ein nicht dargestellter Schlauch angeschlossen werden kann, der mit dem Katheter (1) in Verbindung steht und über eine Rollenpumpe geführt ist, um in der oben beschriebenen Weise gesteuert Blutproben entnehmen zu können.

Der Kolbenschieber (25) enthält einen vom Anschlußstutzen (27) ausgehenden, sich in Längsrichtung des Kolbenschiebers erstreckenden Kanal (28), der in einem radialen Arm (29) endet. Dieser radiale Arm (29) stellt entsprechend der Position des Kolbenschiebers (25) im Zylinder (24) eine Verbindung zu einem von mehreren Anschlußstutzen (30 bis 36) des Zylinders (24) her, an die einzelne nicht dargestellte Behältnisse zur Aufnahme des herangeführten Blutes auswechselbar angeschlossen werden können. Beispielsweise wird an den Anschlußstutzen (30) ein Sammelbehältnis für Blutabfälle angeschlossen, während an die Anschlußstutzen (31 bis 36) insgesamt sechs separate Behältnisse für die Aufnahme einzelner Blutproben auswechselbar angeschlossen werden können.

Mit einem derartigen Ventil ist es möglich, die dem Patienten entnommenen Blutproben einzeln aufzufangen, so daß ein Trennmittel nicht benötigt wird. Die zwischen den einzelnen für die Analyse vorgesehenen Blutproben in der Vorrichtung befindlichen verunreinigten Blutmengen werden durch den Anschlußstutzen (30) als Abfall immer wieder in das daran angeschlossene Behältnis geleitet.

## Patentansprüche

1. Vorrichtung zum Abnehmen von Blutproben in zeitlichen Abständen nacheinander für die Diagnose von Körperfunktionen eines Patienten durch Analysieren einzelner Blutproben, mit einem ständig mit dem Patienten zu verbindenden Katheter (1), einem an den Katheter (1) angeschlossenen flexiblen Schlauch (3), eine nach einem vorgegebenen Programm automatisch ein- und auszuschaltenden Pumpe (9), wobei die Pumpe (9) eine auf den flexiblen Schlauch (3) einwirkende Rollenpumpe ist, wenigstens einem mit dem Katheter (1) über ein umschaltbares Steuerventil (2) und über den Schlauch (3) verbundenen Sammelbehältnis (3a) für die abgenommenen Blutproben (21), wobei das Steuerventil (2) in steuerbarer Folge das Sammelbehältnis (3a) abwechselnd mit dem Katheter (1) und einem Vorratsbehältnis (7) für inertes Trennmittel und/oder Antigerinnungsmittel verbindet, wobei der Schlauch (3) mit dem Katheter (1) und dem Sammelbehältnis (3a) auswechselbar ist und daß zum Steuern des Steuerventils (2) und der Rollenpumpe (9) in der Vorrichtung ein individuell programmierbarer Mikrocomputer (8) vorgesehen ist.

2. Vorrichtung nach Anspruch 1, dadurch gekennzeichnet, daß das Sammelbehältnis (3a) als flexibler Schlauch ausgebildet ist und auf eine Rolle (5) aufwickelbar ist.

3. Vorrichtung nach Anspruch 1 oder 2, dadurch gekennzeichnet, daß das schlauchförmige Sammelbehältnis (3a) aus durchsichtigem flexiblen Material besteht.

4. Vorrichtung nach Anspruch 1, dadurch gekennzeichnet, daß das Steuerventil (2) ein Schieberventil ist.

5. Vorrichtung nach Anspruch 4, dadurch gekennzeichnet, daß der Schieber (13) des Steuerventils (2) elektromagnetisch steuerbar ist.

6. Vorrichtung nach Anspruch 1, dadurch gekennzeichnet, daß sie ein umschaltbares Steuerventil (23) aufweist, das in steuerbarer Folge den Katheter (1) nacheinander mit einer Anzahl von Behältnissen zur Aufnahme einzelner Blutproben und von Blutabfällen verbindet, wobei das Steuerventil (23) ein Schieberventil mit einem Einlaß (27) und einer Anzahl separater Auslässe (30 bis 36) ist.

## Claims

1. Device for taking blood samples one after another at spaced time intervals for the diagnosis of body functions of a patient by analysing individual blood samples, comprising a catheter (1) for permanent connection to the patient, a flexible hose (3) connected to said catheter (1), a pump (9) which is switched on and off automatically according to a predetermined program, the pump (9) being a roller pump acting on the flexible hose (3), at least one collecting container (3a) for taken blood samples (21) connected to said catheter (1) through a change-over control valve (2) and through said hose (3), said control valve (2) connecting said collecting container (3a) in controlled sequence alternatively to said catheter (1) and to a reservoir container (7) for an inert separating medium and/or anticoagulant, said hose (3) together with said catheter (1) and said collecting container (3a) being interchangeable, and an individually programmable microcomputer (8) being provided for controlling said control valve (2) and said roller pump (9) in the device.

2. Device according to claim 1, characterized in that said collecting container (3a) is in the form of a flexible hose and to be wound onto a drum (5).

3. Device according to claim 1 or 2, characterized in that said hose-like collecting container (3a) consists of transparent flexible material.

4. Device according to claim 1, characterized in that said control valve (2) is a slide valve.

5. Device according to claim 4, characterized in that the slide (13) of said control valve (2) is controlled electromagnetically.

6. Device according to claim 1, characterized in that it comprises a change-over control valve (23) connecting said catheter (1) in controlled sequence by turns to a plurality of containers for receiving individual blood samples and blood waste, said control valve (23) being a slide valve having an inlet (27) and a plurality of separate outlets (30 to 36).

## Revendications

1. Dispositif pour prélever des échantillons sanguins à des intervalles de temps successifs pour le diagnostic des fonctions corporelles d'un malade par analyse des divers échantillons sanguins, avec un cathéter (1) à relier constamment au malade, un tuyau flexible (3) fixé au cathéter (1), une pompe (9) à connecter et à déconnecter automatiquement selon un programme préétabli où la pompe est une pompe à cylindre agissant sur le tuyau flexible (3), au moins un récipient de collecte (3a) relié au cathéter (1) par l'intermédiaire d'une soupape de manœuvre commutable (2) et du tuyau (3) pour les échantillons sanguins prélevés (21) où la soupape de manœuvre (2) relie en une succession réglable le récipient de collecte (3a) alternativement au cathéter (1) et à un récipient de réserve (7) pour produit de séparation inerte et/ou agent anti-coagulant, où le tuyau (3) est interchangeable avec le cathéter (1) et le récipient de collecte (3a), et caractérisé en ce que pour la commande de la soupape de manœuvre (2) et de la pompe à cylindre (9) est prévu dans le dispositif un micro-ordinateur (8) programmable individuellement.

2. Dispositif selon la revendication 1, caractérisé en ce que le récipient de collecte (3a) est construit comme un tuyau flexible et est enroulable sur un rouleau (5).

3. Dispositif selon la revendication 1 ou 2, caractérisé en ce que le récipient de collecte en forme de tuyau (3a) se compose d'une matière flexible transparente.

4. Dispositif selon la revendication 1, caractérisé en ce que la soupape de manœuvre (2) est une soupape à coulisse.

5. Dispositif selon la revendication 4, caractérisé en ce que le tiroir (13) de la soupape de manœuvre (2) est commandable par un moyen électro-magnétique.

6. Dispositif selon la revendication 1, caractérisé en ce qu'il présente une soupape de manœuvre commutable (23) qui relie dans une succession réglable le cathéter (1) successivement avec un certain nombre de récipients pour prélever divers échantillons sanguins et sous-produits du sang, où la soupape de manœuvre (23) est une soupape de commande avec une entrée (27) et un certain nombre de sorties séparées (30 à 36).

FIG.1

FIG.2

FIG.3a

FIG.3b

FIG.4

FIG.5